# EUROPEAN PATENT SPECIFICATION

(11) **EP 4 543 281 B1**
(45) Date of publication and mention of the grant of the patent: **20.05.2026**
(21) Application number: 22785925.3
(22) Date of filing: 06.09.2022
(51) Int. Cl.: A61B 3/135, A61B 3/00

(54) **SLIT LAMP MICROSCOPE SPATIAL LIGHT MODULATOR AND LOW-PASS FILTERING**
RÄUMLICHER LICHTMODULATOR FÜR SPALTLAMPENMIKROSKOP UND TIEFPASSFILTERUNG
MODULATEUR SPATIAL DE LUMIÈRE DE MICROSCOPE À LAMPE À FENTE ET FILTRAGE PASSE-BAS

(43) Date of publication of application: 30.04.2025
(73) Proprietor: HAAG-STREIT AG, 3098 Köniz (CH)
(72) Inventor: BREITENSTEIN, Jörg, 3052 Zollikofen (CH); DELLAGIACOMA, Claudio, 3098 Köniz (CH); ROBLEDO, Lucio, 3013 Bern (CH)
(74) Representative: E. Blum & Co. AG
(86) International application number: PCT/EP2022/074715
(87) International publication number: WO 2024/051923

(56) References cited:
- WO-A1-2021/144001
- US-A1- 2015 157 198

## Description

### Technical Field

The invention relates to a slit lamp microscope, i.e. to an ophthalmic microscope that is adapted to illuminate a patient's eye with a slit-shaped or otherwise structured illumination field and to view the eye through a microscope device. The structured illumination is generated by means of an electronically controlled spatial light modulator.

### Background Art

Classically, the illumination device of a slit lamp microscope comprises a slit-aperture and illumination imaging optics adapted to project the slit-aperture into the object plane.

WO 2021144000 describes a slit lamp microscope where the slit-aperture is replaced by a high-resolution spatial light modulator. This type of slit lamp microscope allows to illuminate the eye with a slit-shaped illumination field or another type of structured light field and to quickly and easily adapt the structure of the illumination to the current requirements.

WO 2021/144001 A1 discloses an ophthalmologic microscope comprising an illumination device (9) for projecting light onto an eye (10) to be observed and a microscope device (8) with a camera (16) to view the eye. The illumination device (9) generates pulsed light and is slaved to the frame rate of the camera (9), which allows to run the camera in free-running mode for achieving a high frame rate. The light is pulsed at least at twice the frame rate of the camera to reduce flicker. The illumination device (9) uses an array of micro-mirrors as spatial light modulator (24), and the mirrors are controlled for a balanced deflection over the frame cycles. WO 2021/144001 does not disclose a low-pass filter attenuating at least one of the spatial frequencies fx and fy in the object plane

US 2015/157198 A1 discloses an ophthalmic illumination system comprising primary light source 210, mirror 220, micro-display projector 270 and beam splitter 265. Primary light source 210 generates light 205 which is directed by mirror 220 toward patient's eye 230. Light 205 strikes patient's eye 230 and is reflected, generating reflected light 240-A. Reflected light 240-B passes through beam-splitter 265 and propagates toward practitioner's eye 290, allowing the practitioner to view structures within patient's eye 230. Micro-display projector 270 may include one of a liquid crystal on silicon (LCoS), digital-micro-mirror device (DMD), 2-D micro-electromechanical systems (MEMS) or 2-D X/Y galvanometer set micro-scanner for generating an image. Micro-display projector 270 also may comprise relay optics (e.g., to illuminate a micro-display with an illumination area dimension matching the micro-display size), and a collimation or projection lens. US 2015/157198 A1 does not disclose a microscope device and a low pass filter.

### Disclosure of the Invention

The problem to be solved by the present invention is to further improve the illumination quality of this type of slit lamp microscope.

This problem is solved by the slit lamp microscope of claim 1. Accordingly, the slit lamp microscope comprises at least the following elements:
i) A microscope device: This device projects an object plane into an eyepiece and/or a camera of the slit lamp microscope. In operation, the patient's eye is placed in the object plane such that it can be viewed through the microscope device.
ii) An illumination device: This device is adapted to generate a structured, adaptable illumination field in the object plane. It comprises the following elements:
   a) At least one light source: The light source generates the radiation to be projected into the object plane.
   b) An electronically controlled spatial light modulator: This modulator is adapted to spatially modulate the light from the light source. It has an array of pixel modulators, such as an array of mirrors or liquid crystal light valves. This array has a spatial frequency fx along a first dimension and a spatial frequency fy along a second dimension, with the two dimensions extending transversally, in particular perpendicularly to each other.
   c) Illumination imaging optics: These optics project the array of pixel modulators towards the object plane, thereby generating a structured light field in the object plane. The structure (e.g. the bounds) of this light field can be defined by setting the spatial light modulator to a desired pattern.

According to the invention, the imaging optics comprises a low-pass filter that attenuates at least one of the spatial frequencies fx and fy in the object plane.

In the absence of such a low-pass filter, the structure of the array of pixel modulators is projected, at least to some degree, into the image plane. As described below, this can generate a perceptible periodic modulation of the light in the bright areas of the object plane with a spatial frequency corresponding to fx and fy, respectively. By providing a low-pass filter, i.e. be reducing the transmission of the spatial frequencies of fx and/or fy into the object plane, this modulation is reduced, which allows to obtain a more homogeneous illumination in the bright areas of the illumination.

In particular, the low-pass filter attenuates the modulation transfer function T between the spatial light modulator and the object plane at the spatial frequency fx much more strongly than at the spatial frequency fx/10, i.e. if T1 is the modulation transfer function of the illumination imaging optics without the low-pass filter and T2 is the modulation transfer function of the illumination imaging optics with the low-pass filter, then
T1(fx)/T2(fx) at least 5 times, in particular at least 10 times larger T1(fx/10)/T2(fx/10).

Advantageously, the same applies for dimension y, i.e. T1(fy)/T2(fy) at least 5 times, in particular at least 10 times larger T1(fy/10)/T2(fy/10).

Advantageously, the low-pass filter is adapted to attenuate the transmission of the spatial frequency fx and/or fy into the object plane by at least a factor 10, in particular by at least a factor 20, as compared to the transmission of a tenth the spatial frequency fx/10 and fy/10, respectively. In other words, e.g. for the first dimension x, if
- T2(fx) is the modulation transfer function of the illumination imaging optics at spatial frequency fx and
- T2(fx/10) is the modulation transfer function of the illumination imaging optics at spatial freqeucy fx/10,
in that case the ratio T2(fx/10) /T2(fx) is larger than 10, in particular larger than 20.

Advantageously, this type of attenuation by a factor of at least 10 is achieved for both spatial frequencies fx and fy.

The low-pass filter may be based on various principles, such an imperfect imaging, time variation of the imaging, and/or beam splitting. These techniques may be used individually or in combination.

In one embodiment, the low-pass filter is structured and adapted to generate, at the object plane, at least two spatially offset images of the spatial light modulator. With two or more such offset images, it becomes possible to provide low-pass filtering along at least one dimension of the array of pixel modulators. To provide low-pass filtering along both dimension x, y of the array, the filter has to generate at least three images mutually offset along at least two different, i.e. non-collinear, directions.

Advantageously, the low-pass filter generates, in the object plane, at least four images of the spatial light modulator with a mutual offset along at least two different (i.e. non-collinear) directions.

In a particularly simple embodiment, the low-pass filter comprises at least one beam splitter in the illumination light path after the spatial light modulator (i.e. between the spatial light modulator and the object plane). This beam splitter may be adapted to generate at least two, in particular at least four, mutually offset light fields, which can then be used to generate the mutually offset images in the object plane.

In addition or alternatively thereto, the low-pass filter may comprise an actuator adapted to move at least one optical element of the illumination imaging optics between at least a first and a second position, thereby generating, at the object plane, at least two spatially offset images. Alternatively or in addition thereto, the actuator may be adapted to move the spatial light modulator between at least a first and a second position in order to generate at least two spatially offset images in the object plane. In both cases case, the microscope comprises a control unit adapted to periodically operate the actuator.

Advantageously, the frequency of the periodic actuation is at least 40 Hz, in particular at least 100 Hz, to generate, for the human observer, the impression of a flicker-free, continuous overlap of the at least two images.

In yet another embodiment, the illumination optics has a Fourier plane corresponding to a Fourier transform of the spatial light modulator, and the low-pass filter comprises a spatial filter in the Fourier plane. This spatial filter at least blocks the areas corresponding to the spatial frequencies fx and/or fy and, advantageously, harmonics thereof.

In a further embodiment, the low-pass filter may be implemented by the illumination imaging optics projecting the image of the spatial light modulator into an image plane that is offset by at least 500 µm from the object plane.

In another embodiment, the low-pass filter may comprise a diffusor, in particular a diffractive diffusor.

### Brief Description of the Drawings

The invention will be better understood and objects other than those set forth above will become apparent when consideration is given to the following detailed description thereof. Such description makes reference to the annexed drawings, wherein:
Fig. 1 shows an embodiment of a slit lamp microscope,
Fig. 2 shows an embodiment of the components of the illumination device,
Fig. 3 shows part of a spatial light modulator,
Fig. 4 shows an idealized image of the part of spatial light modulator of Fig. 3 at the object plane for the mirrors in the first position (with dark areas representing the illuminated regions),
Fig. 5 shows, qualitatively, the frequency transmission function of the illumination imaging optics with the low-pass filter,
Fig. 6 shows a first embodiment of a low-pass filter,
Fig. 7 shows an image corresponding to the one of Fig. 4 but with the low-pass filter in place, and
Figs. 8 and 9 show Fourier filters optimized for two different wavelengths.

### Modes for Carrying Out the Invention

### Overview

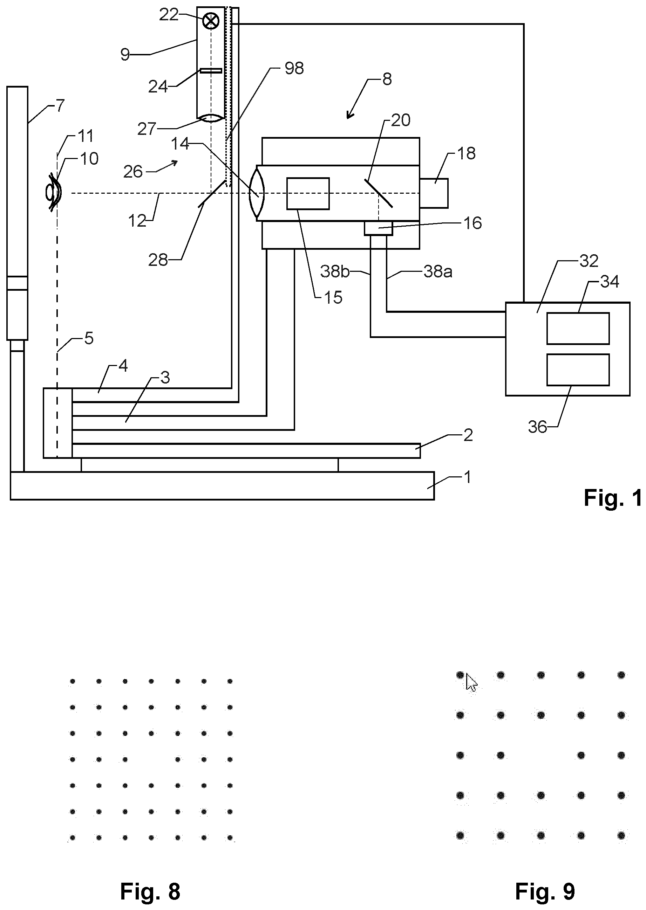

Fig. 1 shows an embodiment of a slit lamp microscope.

The microscope has a base 1 resting e.g. on a desk, a translationally displaceable stage 2 mounted to base 1, a first arm 3, and a second arm 4.

Stage 2 can be linearly displaced along two horizontal directions in respect to base 1.

The arms 3 and 4 are mounted to stage 2 and pivotal about a common vertical pivot axis 5.

The device may further include a headrest 7 mounted to base 1 for receiving the patient's head.

Arm 3 carries a microscope device 8, and arm 4 carries an illumination device 9, the so-called "slit lamp".

Microscope device 8 has an optical axis 12. It comprises microscope optics 14, 15, such as an objective 14 and zoom optics 15, which e.g. project an image of eye 10 onto a camera 16 and/or into an eyepiece 18. A beam splitter 20 or a flip-mirror may be provided to spilt light between these components.

Illumination device 9 is pivotal about pivot axis 5. It is adapted to project a structured light beam onto the eye 10 to be examined. It comprises a light source 22, a spatial light modulator 24, and illumination imaging optics 26 comprising at least one lens 27.

Light source 22 can e.g. comprise several units emitting different wavelengths, e.g. in the red, green, blue, and infrared range of the optical spectrum. These units may be controlled separately in order to change the color of light source 22.

Advantageously, the light source 22 comprises at least one LED and/or semiconductor laser. This type of light sources are advantageous because LEDs and semiconductor lasers can be pulsed rapidly and precisely.

Illumination imaging optics 26 projects the light from modulator 24 onto e.g. the anterior surface of eye 10, e.g. via a deflection mirror 28 mounted to arm 4. The anterior surface of eye 10 is assumed to be located at an object plane 11, which is the optically conjugate plane of spatial light modulator 24 in respect to illumination imaging optics 26. Note that, as described in the section "Defocusing" below, in one embodiment, object plane 11 is not exactly in the optically conjugate plane of the spatial light modulator 24.

Pivot axis 5 lies in object plane 11 in order to keep the center of the image in focus while rotating one or both of the arms 3, 4.

Illumination device 9 can be arranged above or below deflection mirror 28.

A control unit 32 controls the components of the microscope. In particular, it may e.g. comprise a microprocessor 34 and a memory 36. Microprocessor 34 is programmed to carry out the steps for controlling the microscope, and memory 36 contains the data and/or instructions to do so.

### Illumination Device

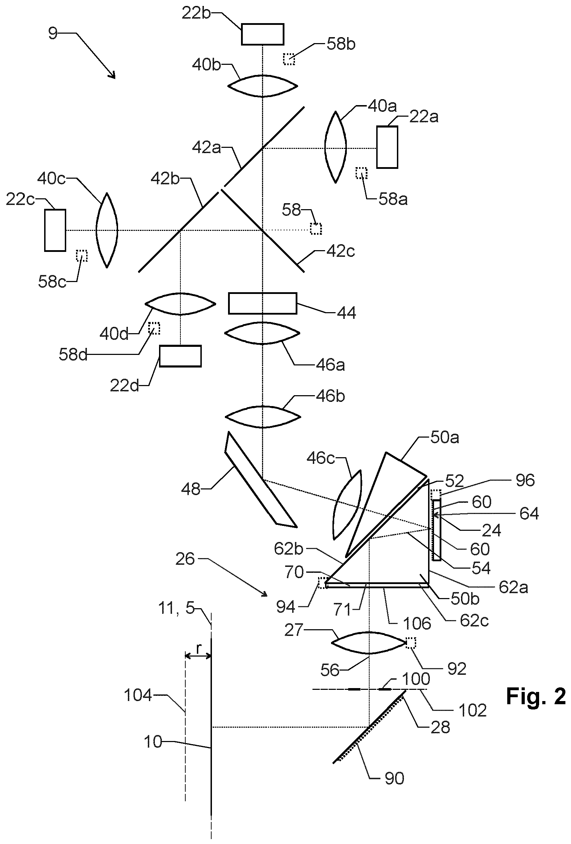

Fig. 2 shows a more detailed embodiment of illumination device 9. It is designed to project an illumination field of defined contours onto the eye 10. The illumination field may e.g. be round, rectangular, or slit-shaped. Even though the illumination device is called a "slit-lamp" herein, the illumination field does not need to be slit-shaped at all. It can take any shape.

In the present embodiment, illumination device 9 comprises four individual light sources 22a - 22d of different spectral emission characteristics, which together form "the light source" 22 of the illumination device. For example, they may include an infrared light source, a red light source, a green light source, and a blue light source. Advantageously, the light sources are LEDs. In particular, each light source may be a single LED.

The light from each light source is substantially collimated by means of collimation optics 40a - 40d.

Three dichroic mirrors 42a, 42b, 42c are used to coaxially combine the light from the light sources 22a - 22d.

The combined illumination light is passed through homogenization optics 44, such as a fly-eye lens array, e.g. as described in US 6507434.

Two cylindrical lenses 46a, 46b, a further lens 46c, and the homogenization optics 44 may together also widen the light beam along one direction, e.g. giving it an elongate cross-section, e.g. having a width-to-height ratio of 16:9, for better matching the typically available form factor of spatial light modulators.

A mirror 48 deflects the light into an assembly of two prisms 50a, 50b with a gap 52 between them.

The light beam passes prism 50a, gap 52, and prism 50b and arrives at spatial light modulator 24.

In the shown embodiment, spatial light modulator 24 is a DMD ("digital micro-mirror device") with a two-dimensional array of individually pivotal micro-mirrors 60 acting as pixel modulators. Control unit 32 is adapted to control the alignment of each micro-mirror 60 between at least a first and a second position.

When a given mirror 60 is in the first position, light from the light source 22 is reflected by the given mirror as shown by line 54, passes a first surface 62a of prism 50b, is totally reflected at a second surface 62b of prism 50b (at gap 52), and exits through a third surface 62c of prism 50b under a direction shown by line 56.

When the given mirror 60 is in the second position, however, light from the light source is reflected by the given mirror and passes first surface 62a, but it is not totally reflected at the second surface 62b. Part of its light exits through surface 62b. The part that is reflected, leaves prism 50b through another surface but not under direction 56, and it will e.g. not pass the aperture of illumination imaging optics 26.

Hence, control unit 32 is able to individually set each pixel (each micro-mirror 60) of spatial light modulator 24 into an on-state and an off-sate, thereby defining the contour and shape of the light field at target 10 (which is shown in Fig. 2 to be located in the object plane 11 of the illumination device).

The light from the pixels of spatial light modulator 24 enters illumination imaging optics 26, which may include one or more lenses. From there, it may pass deflection mirror 28 to arrive at object plane 11.

The illumination imaging optics projects spatial light modulator 24 onto target 10, i.e. target 10 is at the object plane 11, which is the conjugate plane, in respect to illumination imaging optics 26, of the plane 64 of spatial light modulator 24.

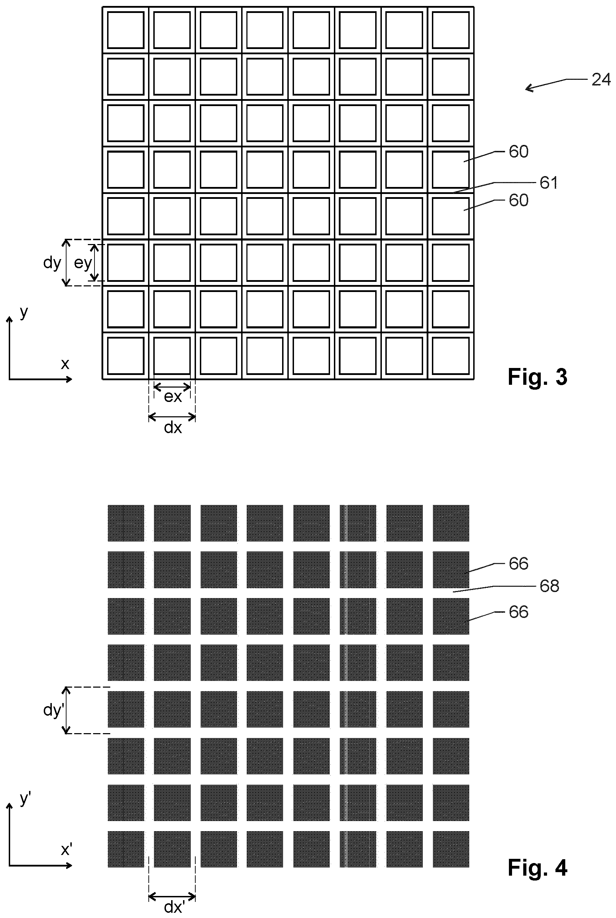

Fig. 3 shows a sub-section of sixty-four mirrors 60 of spatial light modulator 24 in the first position (i.e. in their on-position). As can be seen, the mirrors 60 form a two-dimensional array extending along first and second dimensions x and y. The spacing of the pixels along x is dx, and along y it is dy. The mirrors 60 have extensions ex and ey, respectively, which are typically smaller than dx and dy, i.e. there are gaps 61 between the mirrors 60 that do not reflect light.

Assuming that all mirrors 60 of the modulator section of Fig. 3 are in their first position, the illumination pattern in object plane 11 generated by these mirrors would look as shown in Fig. 4, assuming that the imaging of illumination imaging optics is substantially perfect. (Note: dark regions of Fig. 4 represent the illuminated parts in object plane 11.)

As can be seen, an array of illuminated regions 66 extending along directions x' and y' is created, which has a spacing dx' along direction x' and dy' along direction y'.

Between the illuminated regions 66, there are non-illuminated regions 68 corresponding to the gaps 61 between the mirrors 60. Hence, the illumination is non-homogenous.

When viewing the eye 10 by means of microscope device 8 at high magnification, this non-homogeneous pattern in the bright areas of object plane 11 can become apparent. This is particularly true for examinations that rely on specular reflections of the light on the eye, e.g. when viewing the front or back surface of the cornea, e.g. for examining the endothelium, or when viewing the front or back side of the natural lens. The non-homogenous illumination also is particularly apparent when recording a sectional view of the eye with narrow-slit illumination.

Even though the artefacts due to the non-homogeneous illumination may be removed from a camera image by means of image processing, such processing may be difficult, in particular if the structure of the illumination interferes with the pixel-structure of the camera, and the processing is impossible when viewing the microscope image directly through the eyepiece. Also, any such processing will potentially reduce the resolution of microscope device 8.

### Low-pass filter

To reduce artefacts due to the inhomogeneous illumination, the illumination device is equipped with a low-pass filter, such as the beam splitter or other means described in the following sections.

Namely, the purpose of this low-pass filter is to suppress (i.e. attenuate) the spatial frequencies fx = 1/dx and/or fy = 1/dy along x and y when imaging spatial light modulator 24 into object plane 11.

In the coordinate system of object plane 11, the spatial frequencies fx and fy of the light modulator correspond to spatial frequencies fx' = 1/dx' and fy' = 1/dy'.

For the following, we define T as the modulation transfer function of illumination imaging optics 26, which is a function of the spatial frequency f at spatial light modulator 24 (see https://en.wikipedia.org/wiki/Optical_transfer_function). f is the spatial frequency of the dimensions x or y. (Note: for simplicity, we assume that both dimensions x and y have the same modulation transfer function T. If not, Tx(f) and Ty(f) may be used.)

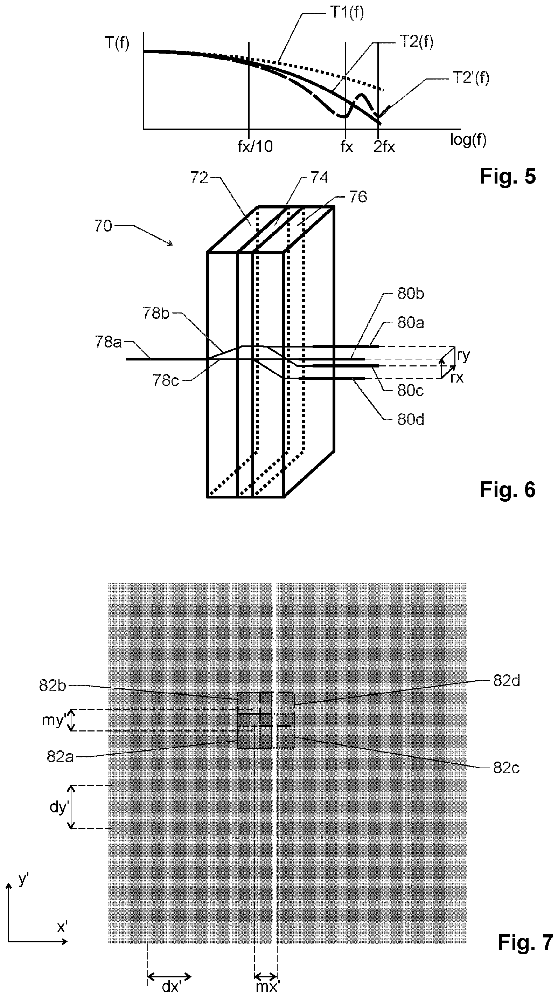

Fig. 5 shows the modulation transfer function T1(f) of illumination imaging optics 26 in the absence of a low-pass filter. As can be seen, T1 decreases for larger frequencies, but at least a residual modulation transfer takes place at frequency fx.

Fig. 5 further shows the modulation transfer function T2(f) of illumination imaging optics 26 when the low-pass filter is present. As can be seen, the low-pass filter generates a strong attenuation at fx but a much smaller attenuation at lower spatial frequencies, e.g. fx/10. Hence, for a given brightness of the illuminated regions of the target at the object plane, illumination inhomogeneities at fx are suppressed.

As mentioned above, advantageously, the attenuation of the modulation transfer function at fx/10 is smaller, by at least a factor 5, in particular at least a factor 10, than at fx, i.e. T1(fx)/T2(fx) is at least 5 times, in particular at least 10 times larger than T1(fx/10)/T2(fx/10).

The same advantageously also applies for fy.

As can also be seen from Fig. 5, the modulation transfer function T2(f) with the low-pass filter should be considerably smaller at fx than at lower frequencies, such as fx/10, for illumination inhomogeneities at fx to be poorly visible. Hence, as mentioned above, the ratio T2(fx/10) /T2(fx) is advantageously larger than 10, in particular larger than 20. The same advantageously also applies for fy.

Fig. 5 shows, under T2'(f) a further variant of the modulation transfer function in the presence of the low-pass filter. In this variant, the modulation transfer function T2'(f) has a local minimum at fx and then goes up again for higher frequencies, e.g. for 1.5·fx, this improves the imaging quality for high-frequency components of the illumination pattern that are not caused by the repetitive structure of the spatial light modulator. However, T2'(f) should advantageously decrease again towards the second harmonic frequency 2·fx.

Hence, more general terms, the modulation transfer function T2(fx) at frequency fx is advantageously smaller, by at least a factor 2, than the modulation transfer function at T2(1.5·fx) at frequency 1.5·fx, i.e. T2(fx) < T2(1.5·fx)/2. In particular T2(1.5·fx) at frequency 1.5·fx is advantageously larger, by at least a factor 2, than T2(2·fx) at frequency 2·fx, i.e. T2(2·fx) < T2(1.5·fx)/2.

Such frequency tuning of the modulation transfer function is best achieved using the techniques of Fourier Filtering as described below.

There are various ways to implement such low-pass filtering, some of which are described in the following sections.

### Beam Splitter

A first embodiment of a low-pass filter is shown in Fig. 6. In this embodiment, the low-pass filter comprises a beam splitter 70. It is arranged in the path of the illumination light after spatial light modulator 24 and before object plane 11, and it is adapted to generate at least two, in particular at least four mutually offset light fields.

In the shown embodiment, beam splitter 70 comprises a first birefringent plate 72, a quarter-wave retarder 74, and a second birefringent plate 76, with the quarter-wave retarder 74 being arranged between the birefringent plates 72, 76.

First birefringent plate 72 has its optic axes arranged advantageously at 45° to its input surface to cause the incoming light 78a to split into a two light fields 78b, 78c of equal intensities. (Here, it is assumed that the incoming light 78a is non-polarized. If it is partially or fully polarized, one or more polarizers and/or wave retarder plates may be used to adapt the polarization to make the light fields 78b, 78c have the same intensities, as known to the skilled person.)

Since the light fields 78b, 78c after first birefringent plate 72 are linearly polarized, quarter-wave retarder 74 is used to transform their polarizations into circular polarizations.

Then, the light fields 78b, 78c enter second birefringent plate 76. Again, second birefringent plate 76 has its optic axes arranged at 45° to its input surface to cause the incoming light fields 78b, 78c to split once more. Second birefringent plate 76 is under a different orientation than first birefringent plate 72 to generate a split into a different direction. Hence, at the output side of beam splitter 70, the incoming light 78a has been split into four mutually offset light fields 80a, 80b, 80c, and 80d.

Advantageously, the four light fields 80a - 80d are mutually offset along rectangular directions rx, ry, which correspond to the dimensions x.

The offset between the light fields along rx and ry is approximately half of the spacing dx and dy, respectively, of the mirrors 80 for the reasons explained below.

Beam splitter 70 may be arranged adjacent to prism 50b, in particular adjacent to first surface 62a or third surface 62c. Advantageously, it is mounted to one of these surfaces, in particular glued thereto. Arranging a transparent filler (such as a glue) between 50b and beam splitter 70 to prevent an air gap between the two parts, is particularly advantageous because it reduces the number of surfaces the light has to traverse.

In the embodiment of Fig. 2, beam splitter 70 is shown to be arranged on third surface 62c, with the transparent filler 71 between the two parts.

Beams splitter 70 acts, as mentioned, as a low-pass filter. In particular, if it is not arranged too close to lens 27, it substantially splits the image of spatial light modulator 24 in object plane 11 (as shown without beam splitter in Fig. 4) into spatially offset images as illustrated in Fig. 7.

In order to illustrate these four images, Fig. 7 shows, under reference numbers 82a - 82d, a single mirror 60 in each of these four images, outlined by differently dashed lines.

As can be seen by comparing Figs. 4 and 7, the illumination pattern of Fig. 7 is more homogeneous than the one of Fig. 4, i.e. the mirror structure is less evident, which gives rise to images of better quality.

The offset of the images along direction x' is mx' and the offset along direction y' is my'. The offsets should be in a range to "blur" the non-illuminated regions 68 of Fig. 4. Hence, advantageously, mx' = dx'/2 and my' = dy'/2. However, if the gaps between the mirrors 60 are not too large, the offsets may deviate from these numbers. Along x', offset mx' is advantageously between 0.2·dx' = 0.2/fx' and 0.8·dx' = 0.8/fx'. Similarly, along y', offset my' is advantageously between 0.2·dy' = 0.2/fy' and 0.8·dy' = 0.8/fy'. Advantageously, mx' is between 0.4/fx' and 0.6/fx' and my' between 0.4/fy' and 0.6/fy'.

Beam splitters based on birefringent plates have been known for a completely different purpose, namely as anti-aliasing filters arranged on camera chips, as e.g. described by Schöberl et al., in Dimensioning of Optical Birefringent Anti-Alias Filters for Digital Cameras, Proc. of 2010 IEEE 17th International Conference on Image Processing, Sept. 26 - 29, Hong Kong. That document mentions stacks of M = 2, M = 3, M = 4 or even more mutually rotated birefringent plates, which can be used in the present context as well.

Hence, in more general terms, the beam splitter 70 advantageously comprises at least two differently oriented birefringent plates 72, 76 and a quarter-wave retarder 74 arranged between each two birefringent plates 72, 76.

Instead of using birefringent beam splitters, the present technique may also use other types of beam splitters generating two or more mutually offset light fields. For example, a semi-transparent mirror can be used to split the light, and the resulting light fields can be redirected and guided by mirrors to be parallel and at the desired offset.

### Actuators

In another class of embodiments, the low-pass filter may comprise one or more actuators.

Such an actuator 90 is shown in dotted lines in Fig. 2 to be arranged at deflection mirror 28 of illumination imaging optics 26. Actuator 90, which may e.g. comprise one or more piezo elements, is adapted to change the tilt angle of deflection mirror 28, thereby offsetting the image of spatial light modulator 24 in object plane 11.

Advantageously, actuator 90 is adapted to tilt deflection mirror 28 about two transversal axes in order to generate, in object plane 11, images offset along both directions x' and y'.

Actuator 90 is periodically operated by control unit 32 to move the image quickly. If such movement is fast enough, e.g. with a period of at least 40 Hz, advantageously at least 100 Hz, several spatially offset images are generated on object plane 11 for a human observer, which again blurs the non-illuminated regions 68 of Fig. 4, thereby generating a more uniform illumination.

Alternatively (or in addition to) moving deflection mirror 28, another part of illumination imaging optics 26 may be moved by the actuator. For example, Fig. 2 shows, another actuator 92 adapted to periodically move lens 27, and another one, at reference number 94, to move prism assembly 50a, 50b or part thereof.

As a further variant, an actuator 96 may be provided to move spatial light modulator 24 along directions x and/or y.

In yet another embodiment as illustrated in Fig. 1, an actuator 98 may be provided to move all of illumination device 9.

To assess if a given actuator-based device is a low-pass filter in the sense of the invention, the image of the spatial light modulator 24 at object plane 11 is advantageously averaged over a period of time to obtain a "subjective image", i.e. the image as perceived by a human observer. Such averaging takes place while the actuator is being periodically operated. The averaging extends advantageously over a period of at least 2.5 ms (corresponding to 40 Hz), in particular over a period of at least 5 ms (corresponding to 20 Hz). Low-pass filtering is then detected by testing if the special frequencies fx' and/or fy' in the subjective image have been suppressed, e.g. by a factor of at least 2, in particular by a factor of at least 5 as described above.

### Fourier Filtering

In yet another embodiment, a filter 100 is placed in the back focal plane (Fourier plane) 102 of lens 27 (see Fig. 2). This plane holds a Fourier transform of the image of spatial light modulator 24. Filter 100 is adapted to suppress the Fourier components corresponding to the spatial frequencies fx and fy as well as, advantageously, their higher harmonics.

For narrow-band illumination, filter 100 may e.g. consist of a simple aperture blocking spatial frequencies above a given threshold, e.g. 0.8 fx and 0.8 fy.

If it is desired to only block fx and fy as well as harmonics thereof, as illustrated by modulation transfer function T2' in Fig. 5, filter 100 may e.g. be calculated from a Fourier transform of the pattern generated by the spatial light modulator (i.e. a pattern as shown in Fig. 4). Such a filter 100 is shown in Fig. 8, where the dark parts indicate parts that block the light.

As known to the skilled person, the Fourier transform in back focal plane 102 is scaled with the wavelength of the light. Hence, if the light source 22 is a broad-band light source, filter 100 can e.g. comprise several superimposed color filters attributed to different wavelengths, with each color filter blocking the spatial frequency components fx, fy (and its harmonics) at a wavelength range centered on its attributed wavelength.

If there are, as shown in the embodiment of Fig. 2, several individual light sources 22a - 22d centered on different wavelengths λi, filter 100 advantageously comprises several wavelength-selective filters that block the spatial frequency fk and its harmonics for the wavelengths λi. For example, Fig. 9 shows the same filter as the one of Fig. 8 but scaled for approximately the double wavelength. For example, the filter of Fig. 9 would (in the dark parts) be absorbing at a first (longer) wavelength λ1 (but not or much less so at λ2), and the filter of Fig. 8 would (in the dark parts) be absorbing at second (shorter) wavelength λ2 (but not or much less so at λ1).

Hence, in more general terms, filter 100 advantageously comprises several wavelength-selective filters that suppress the spatial frequency fk and, advantageously, harmonics thereof, for several different wavelengths λi, with the filters comprising light-blocking structures scaled in size with the different wavelengths λi. Advantageously, in this case, illumination device 9 comprises several light sources having different emission spectra with maximum emissions at the wavelengths λi.

### Defocusing

In another embodiment, the low-pass filter may be implemented by intentionally de-focusing illumination imaging optics 26. For example, lens 27 may be dimensioned to project the image of spatial light modulator not into object plane 11 but into an image plane 104 (as illustrated in Fig. 2) that is offset by an offset r of at least 500 µm from the object plane 11.

As mentioned above, illumination device 9 is pivotal about vertical pivot axis 5, with pivot axis 5 lying in object plane 11. Hence, such de-focusing implies that image of the spatial light modulator 24 comes to lie before or behind pivot axis 5.

As shown in Fig. 2, object plane 11 is best arranged between microscope device 8 and image plane 104, i.e. image plane 104 is located, as seen from microscope device 8, behind object plane 11. This is advantageous because, when microscope device 8 is adjusted (as it is common) for the user to focus their eyes to infinity in order to sharply see objects in object plane 11, it becomes harder for the user to focus on image plane 104, i.e. it is less likely that the user "accidentally" focuses on image plane 104.

In another embodiment, such defocusing may be implemented by introducing spherical aberrations in lens 27 or in other parts of imaging optics 26.

Spherical aberration, also higher order spherical aberrations, are field position and rotation independent aberrations (such as defocus) and can be used to blur the image of the spatial light modulator intentionally and homogeneously in the whole object plane, thereby forming a low-pass filter. In a similar manner, other aberrations, such as astigmatism, act as low-pass filter. Advantageously, the combination of the classical and higher order aberrations can be used to create the desired low-pass filter.

### Diffusors

In yet another embodiment, a diffusor 106 arranged between spatial light modulator 24 and object plane 11 may be used as a low-pass filter. Such a diffusor may e.g. be arranged, same as beam splitter 70, at first or third surface 62a, 62c of prism 50b. Alternatively, it may e.g. also be arranged in the Fourier plane.

Advantageously, diffusor 106 is a diffractive diffusor, i.e. a diffusor that uses diffraction for diffusing the light.

### Notes

In the embodiments above, the low-pass filter is adapted to suppress the spatial frequencies fx and fy along x as well as y. Alternatively, the low-pass filter may also be designed to suppress only one of these spatial frequencies, in particular if the illumination device is designed such that, at object plane 11, one of the corresponding frequencies fx' and fy' is much larger than the other one. In that case, only the smaller frequency may have to be suppressed because the larger frequency may be beyond the resolution of microscope device 8.

The various embodiments of low-pass filters described above may also be combined, e.g. with a beam splitter for one direction x and an actuator for another direction y.

In the embodiments above, the light source and the spatial light modulator are separate elements. Alternatively, they may also be formed by a single device having an array of light sources, with each light source defining the illumination in one pixel.

While there are shown and described presently preferred embodiments of the invention, it is to be distinctly understood that the invention is not limited thereto but may be otherwise variously embodied and practiced within the scope of the following claims.

## Claims

1. A slit lamp microscope comprising
i) a microscope device (8) projecting an object plane (11) into at least one of an eyepiece (18) or a camera (16) and
ii) an illumination device (9) having
a) at least one light source (22),
b) an electronically controlled spatial light modulator (24) comprising an array of pixel modulators extending along first and second dimensions (x, y), wherein said pixel modulators have spatial frequencies fx and fy along the first and second dimensions (x, y), and
c) illumination imaging optics (26) projecting the array towards the object plane (11), **characterized in that** the illumination device (9) comprises a low-pass filter (70, 90 - 100, 106) attenuating at least one of the spatial frequencies fx and fy in the object plane (11).

2. The microscope of claim 1 wherein, if T1 is a modulation transfer function of the illumination imaging optics (26) between the spatial light modulator (24) and the object plane (11) without the low-pass filter (70, 90 - 100, 106) and T2 is a modulation transfer function of the illumination imaging optics (26) between the spatial light modulator (24) and the object plane (11) with the low-pass filter (70, 90 - 100, 106), T1(fx)/T2(fx) at least 5 times, in particular at least 10 times larger T1(fx/10)/T2(fx/10),
and in particular T1(fy)/T2(fy) is also at least 5 times, in particular at least 10 times larger T1(fy/10)/T2(fy/10).

3. The microscope of any of the preceding claims wherein, if T2 is a modulation transfer function of the illumination imaging optics (26) between the spatial light modulator (24) and the object plane (11) with the low-pass filter (70, 90 - 100, 106), T2(fx/10) /T2(fx) is larger than 10, in particular larger than 20.

4. The microscope of claim 3 wherein T2(fx) < T2(1.5·fx)/2, and in particular wherein T2(2·fx) < T2(1.5-fx)/2.

5. The microscope of any of the preceding claims wherein the low-pass filter (70, 90 - 96, 100, 106) is adapted to generate, at the object plane (11), at least two spatially offset images (82a - 82d) of the spatial light modulator (24).

6. The microscope of claim 5 wherein, at the object plane (11), the images (82a - 82d) of the spatial light modulator (24) have an offset mx', along the imaged first dimension, between 0.2/fx' and 0.8/fx', in particular between 0.4/fx' and 0.6/fx', with fx' and fy' being the spatial frequencies of the array in the images at the image plane.

7. The microscope of any of the claims 5 or 6 wherein the low-pass filter (70, 90 - 100, 106) is adapted to generate, at the object plane (11), at least four spatially offset images (82a - 82d) of the spatial light modulator (24).

8. The microscope of any of the preceding claims wherein said low-pass filter (70, 90 - 100, 106) comprises at least one beam splitter (70) after the spatial light modulator (24), wherein the beams splitter (70) is adapted to generate at least two mutually offset light fields (80a - 80d).

9. The microscope of claim 8 wherein the beam splitter (70) is adapted to generate at least four mutually offset light fields (80a - 80d).

10. The microscope of claim 9 wherein the beams splitter (70) comprises at least two differently oriented birefringent plates (72, 76) and a quarter-wave retarder (74) arranged between each two birefringent plates (72, 76).

11. The microscope of any of the claims 9 or 10 wherein illumination device (9) comprises
an array of individually pivotal mirrors (60), with each mirror (60) having a first and a second position,
a prism (50b) having first, second, and third surfaces (62a, 62b, 62c), wherein, for a given mirror (60) in the first position, light from the light source (22) is reflected by the given mirror (60), passes the first surface (62a), is totally reflected at the second surface (62b), and exits through the third surface (62c), while, for the given mirror (60) in the second position, the light from the light source (22) is reflected by the given mirror (60), passes the first surface (62a), and is not totally reflected at the second surface (62b),
and wherein the beam splitter (70) is arranged adjacent to said prism (50b).

12. The microscope of claim 11 wherein the beam splitter (70) is arranged on said first or third surface (62a, 62c), in particular said third surface (62c).

13. The microscope of claim 12 wherein the beam splitter (70) is connected to the first or third surface (62a, 62c) via a transparent filler (71).

14. The microscope of any of the preceding claims wherein said low-pass filter (70, 90 - 100, 106) comprises an actuator (90 - 100) adapted to move
at least one optical element of the illumination imaging optics (26) and/or
the spatial light modulator (24),
between at least a first and a second position, thereby generating, at the object plane (11), at least two spatially images (82a - 82d), and wherein the microscope comprises a control unit (32) adapted to periodically operate the actuator (90 - 100).

15. The microscope of claim 14 wherein the illumination imaging optics (26) comprises at least one deflection mirror (28) deflecting light from the spatial light modulator (24) towards said object plane (11), wherein the actuator (90) is adapted to change at least one tilt angle of the deflection mirror (28).

16. The microscope of any of the preceding claims wherein said illumination optics has a Fourier plane (102) holding a Fourier transform of the spatial light modulator (24), wherein said low-pass filter (100) suppresses Fourier-components of the image of the spatial light modulator (24) in the Fourier plane (102).

17. The microscope of claim 16 wherein said low-pass filter (100) comprises several wavelength-selective filters that suppress the spatial frequency fk and, advantageously, harmonics thereof, for several different wavelengths λi, with the filters comprising blocking structures scaled with the different wavelengths λi.

18. The microscope of claim 17 wherein the illumination device (9) comprises several light sources (22a - 22d) having different emission spectra with maximum emissions at the wavelengths λi.

19. The microscope of any of the preceding claims
wherein said illumination device (9) is pivotal about a pivot axis (5) lying in said object plane (11) and
wherein the low-pass filter (70, 90 - 100, 106) is implemented by said illumination imaging optics (26) projecting the image of the spatial light modulator (24) into an image plane (104) that is offset by at least 500 µm from the object plane (11).

20. The microscope of claim 19 wherein the object plane (11) is located between the microscope device (8) and the image plane (104).

21. The microscope of any of the preceding claims wherein the low-pass filter (70, 90 - 100, 106) comprises a diffusor (106), in particular a diffractive diffusor.

## Patentansprüche

1. Spaltlampenmikroskop, bestehend aus
i) ein Mikroskop (8), das eine Objektebene (11) in mindestens ein Okular (18) oder eine Kamera (16) projiziert und
ii) eine Beleuchtungsvorrichtung (9) mit
a) mindestens einer Lichtquelle (22),
b) einem elektronisch gesteuerten räumlichen Lichtmodulator (24) mit einer Anordnung von Pixelmodulatoren, die sich entlang einer ersten und einer zweiten Dimension (x, y) erstrecken, wobei die Pixelmodulatoren Ortsfrequenzen fx und fy entlang der ersten und zweiten Dimension (x, y) besitzen, und
c) eine Beleuchtungs-Abbildungs-Optik (26), die das Array auf die Objektebene (11) projiziert.
**dadurch gekennzeichnet, dass** die Beleuchtungsvorrichtung (9) einen Tiefpassfilter (70. 90 - 100. 106) aufweist, der mindestens eine der Ortsfrequenzen fx und fy in der Objektebene (11) dämpft.

2. Mikroskop nach Anspruch 1, wobei, wenn T1 ein Modulationsübertragungsfunktion der Beleuchtungs-Abbildungs-Optik (26) zwischen dem räumlichen Lichtmodulator (24) und der Objektebene (11) ohne den Tiefpassfilter (70. 90 - 100. 106) ist und T2 einer Modulationsübertragungsfunktion der Beleuchtungs-Abbildungs-Optik (26) zwischen dem räumlichen Lichtmodulator (24) und der Objektebene (11) mit dem Tiefpassfilter (70. 90 - 100. 106), dann ist T1(fx)/T2(fx) mindestens 5-mal, insbesondere mindestens 10-mal grösser als T1(fx/10)/T2(fx/10) ist.
und insbesondere wobei T1(fy)/T2(fy) auch mindestens 5-mal, insbesondere mindestens 10-mal grösser ist als T1(fy/10)/T2(fy/10).

3. Mikroskop nach einem der vorangehenden Ansprüche, wobei, wenn T2 eine Modulationsübertragungsfunktion der Beleuchtungs-Abbildungs-Optik (26) zwischen dem räumlichen Lichtmodulator (24) und der Objektebene (11) mit dem Tiefpassfilter (70. 90 - 100. 106) ist, T2(fx/10)/T2(fx) grösser als 10. insbesondere grösser als 20 ist.

4. Mikroskop nach Anspruch 3, wobei T2(fx) < T2(1.5·fx)/2, und insbesondere wobei T2(2·fx) < T2(1.5·fx)/2.

5. Mikroskop nach einem der vorangehenden Ansprüche, wobei der Tiefpassfilter (70. 90 - 96, 100. 106) so ausgelegt ist, dass er in der Objektebene (11) mindestens zwei räumlich versetzte Bilder (82a - 82d) des räumlichen Lichtmodulators (24) erzeugt.

6. Mikroskop nach Anspruch 5, bei dem die Bilder (82a - 82d) des räumlichen Lichtmodulators (24) in der Objektebene (11) einen Versatz mx' entlang der abgebildeten ersten Dimension zwischen 0.2/fx ' und 0.8/fx ', insbesondere zwischen 0.4/fx ' und 0.6/fx ' aufweisen, wobei fx ' und fy ' die Ortsfrequenzen des Arrays in den Bildern in der Bildebene sind.

7. Mikroskop nach einem der Ansprüche 5 oder 6, wobei der Tiefpassfilter (70. 90 - 100. 106) so ausgelegt ist, dass er in der Objektebene (11) mindestens vier räumlich versetzte Bilder (82a - 82d) des räumlichen Lichtmodulators (24) erzeugt.

8. Mikroskop nach einem der vorangehenden Ansprüche, wobei der Tiefpassfilter (70. 90 - 100. 106) nach dem räumlichen Lichtmodulator (24) mindestens einen Strahlteiler (70) aufweist, der so ausgelegt ist, dass er mindestens zwei zueinander versetzte Lichtfelder (80a - 80d) erzeugt.

9. Mikroskop nach Anspruch 8, wobei der Strahlteiler (70) so ausgelegt ist, dass er mindestens vier zueinander versetzte Lichtfelder (80a - 80d) erzeugt.

10. Mikroskop nach Anspruch 9, wobei der Strahlteiler (70) mindestens zwei unterschiedlich orientierte doppelbrechende Platten (72, 76) und einen zwischen je zwei doppelbrechenden Platten (72, 76) angeordneten Viertelwellenverzögerer (74) umfasst.

11. Mikroskop nach einem der Ansprüche 9 oder 10. wobei die Beleuchtungsvorrichtung (9) Folgendes umfasst
eine Anordnung von einzeln drehbaren Spiegeln (60), wobei jeder Spiegel (60) eine erste und eine zweite Position hat,
ein Prisma (50b) mit einer ersten, zweiten und dritten Oberfläche (62a, 62b, 62c), wobei bei einem gegebenen Spiegel (60) in der ersten Position das Licht der Lichtquelle (22) vom gegebenen Spiegel (60) reflektiert wird, die erste Oberfläche (62a) passiert, an der zweiten Oberfläche (62b) totalreflektiert wird und durch die dritte Oberfläche (62c) austritt, während bei dem gegebenen Spiegel (60) in der zweiten Position das Licht der Lichtquelle (22) vom gegebenen Spiegel (60) reflektiert wird, die erste Oberfläche (62a) passiert und an der zweiten Oberfläche (62b) nicht totalreflektiert wird,
und wobei der Strahlteiler (70) beim Prisma (50b) angeordnet ist.

12. Mikroskop nach Anspruch 11, wobei der Strahlteiler (70) auf der ersten oder dritten Oberfläche (62a, 62c), insbesondere auf der dritten Oberfläche (62c), angeordnet ist.

13. Mikroskop nach Anspruch 12, wobei der Strahlteiler (70) über ein transparentes Füllmaterial (71) mit der ersten oder dritten Oberfläche (62a, 62c) verbunden ist.

14. Mikroskop nach einem der vorangehenden Ansprüche, wobei der Tiefpassfilter (70. 90 - 100. 106) einen Aktor (90 - 100) umfasst, der ausgestaltet ist zum Bewegen
mindestens eines optischen Element der Beleuchtungs-Abbildungs-Optik (26) und/oder
des räumlichen Lichtmodulators (24),
zwischen mindestens einer ersten und einer zweiten Position, wodurch in der Objektebene (11) mindestens zwei räumliche Bilder (82a - 82d) erzeugt werden, wobei das Mikroskop eine Steuereinheit (32) umfasst, die so ausgelegt ist, dass sie den Aktor (90 - 100) periodisch betätigt.

15. Mikroskop nach Anspruch 14, wobei die Beleuchtungs-Abbildungs-Optik (26) mindestens einen Ablenkspiegel (28) umfasst, der Licht vom räumlichen Lichtmodulator (24) auf die Objektebene (11) ablenkt, wobei der Aktor (90) dazu ausgestaltet ist, mindestens einen Neigungswinkel des Ablenkspiegels (28) zu ändern.

16. Mikroskop nach einem der vorangehenden Ansprüche, wobei die Beleuchtungsoptik eine Fourier-Ebene (102) aufweist, die eine Fourier-Transformierte des räumlichen Lichtmodulators (24) enthält, wobei der Tiefpassfilter (100) in der Fourier-Ebene (102) Fourier-Komponenten des Bildes des räumlichen Lichtmodulators (24) unterdrückt.

17. Mikroskop nach Anspruch 16, wobei der Tiefpassfilter (100) mehrere wellenlängenselektive Filter umfasst, die die Ortsfrequenz fk und vorteilhafterweise deren Harmonische, für mehrere verschiedene Wellenlängen λi unterdrücken, wobei die Filter Sperrstrukturen umfassen, die mit den verschiedenen Wellenlängen λi skaliert sind .

18. Mikroskop nach Anspruch 17, wobei die Beleuchtungsvorrichtung (9) mehrere Lichtquellen (22a - 22d) mit unterschiedlichen Emissionsspektren und Emissionsmaxima bei den Wellenlängen λi umfasst.

19. Mikroskop eines der vorangehenden Ansprüche
wobei die Beleuchtungsvorrichtung (9) um eine in der Objektebene (11) liegende Drehachse (5) schwenkbar ist und
wobei der Tiefpassfilter (70. 90 - 100. 106) dadurch implementiert ist, dass die Beleuchtungs-Abbildungs-Optik das Bild des räumlichen Lichtmodulators (24) in eine Bildebene (104) projiziert, die um mindestens 500 µm von der Objektebene (11) versetzt ist.

20. Mikroskop nach Anspruch 19, wobei sich die Objektebene (11) zwischen der Mikroskopvorrichtung (8) und der Bildebene (104) befindet.

21. Mikroskop nach einem der vorangehenden Ansprüche, wobei der Tiefpassfilter (70. 90 - 100. 106) einen Diffusor (106), insbesondere einen diffraktiven Diffusor, umfasst.

## Revendications

1. Un microscope à lampe à fente comprenant
i) un dispositif microscopique (8) projetant un plan objet (11) dans au moins l'un d'un oculaire (18) ou d'une caméra (16) et
ii) un dispositif d'éclairage (9) comprenant
a) au moins une source de lumière (22),
b) un modulateur spatial de lumière à commande électronique (24) comprenant un réseau de modulateurs de pixels s'étendant le long d'une première et d'une deuxième dimension (x, y), dans lequel lesdits modulateurs de pixels ont des fréquences spatiales fx et fy le long des première et deuxième dimensions (x, y), et
c) une optique d'imagerie d'éclairage (26) projetant le réseau vers le plan objet (11),
**caractérisé en ce que** le dispositif d'éclairage (9) comprend un filtre passe-bas (70, 90 - 100, 106) atténuant au moins l'une des fréquences spatiales fx et fy dans le plan objet (11).

2. Le microscope selon la revendication 1, dans lequel, si T1 est une fonction de transfert de modulation de l'optique d'imagerie d'éclairage (26) entre le modulateur spatial de lumière (24) et le plan objet (11) sans le filtre passe-bas (70, 90 - 100, 106) et T2 est une fonction de transfert de modulation de l'optique d'imagerie d'éclairage (26) entre le modulateur spatial de lumière (24) et le plan objet (11) avec le filtre passe-bas (70, 90 - 100, 106), T1(fx)/T2(fx) au moins 5 fois, en particulier au moins 10 fois plus grand que T1(fx/10)/T2(fx/10),
et en particulier T1(fy)/T2(fy) est également au moins 5 fois, en particulier au moins 10 fois plus grand que T1(fy/10)/T2(fy/10).

3. Le microscope selon l'une quelconque des revendications précédentes, dans lequel, si T2 est une fonction de transfert de modulation de l'optique d'imagerie d'éclairage (26) entre le modulateur spatial de lumière (24) et le plan objet (11) avec le filtre passe-bas (70, 90 - 100, 106), T2(fx/10) /T2(fx) est supérieur à 10, en particulier supérieur à 20.

4. Le microscope selon la revendication 3, dans lequel T2(fx)<T2(1,5·fx)/2, et en particulier dans lequel T2(2·fx)<T2(1,5·fx)/2.

5. Le microscope selon l'une quelconque des revendications précédentes, dans lequel le filtre passe-bas (70, 90 - 96, 100, 106) est adapté pour générer, au niveau du plan objet (11), au moins deux images décalées spatialement (82a - 82d) du modulateur spatial de lumière (24).

6. Le microscope selon la revendication 5, dans lequel, au niveau du plan objet (11), les images (82a - 82d) du modulateur spatial de lumière (24) ont un décalage mx', le long de la première dimension imagée, compris entre 0,2/fx' et 0,8/fx', en particulier entre 0,4/fx' et 0,6/fx', fx' et fy' étant les fréquences spatiales du réseau dans les images au niveau du plan image.

7. Le microscope selon l'une quelconque des revendications 5 ou 6, dans lequel le filtre passe-bas (70, 90 - 100, 106) est adapté pour générer, au niveau du plan objet (11), au moins quatre images décalées spatialement (82a - 82d) du modulateur spatial de lumière (24).

8. Le microscope selon l'une quelconque des revendications précédentes, dans lequel ledit filtre passe-bas (70, 90 - 100, 106) comprend au moins un séparateur de faisceau (70) après le modulateur spatial de lumière (24), dans lequel le séparateur de faisceau (70) est adapté pour générer au moins deux champs lumineux mutuellement décalés (80a - 80d).

9. Le microscope selon la revendication 8, dans lequel le séparateur de faisceau (70) est adapté pour générer au moins quatre champs lumineux mutuellement décalés (80a - 80d).

10. Le microscope selon la revendication 9, dans lequel le séparateur de faisceaux (70) comprend au moins deux plaques biréfringentes (72, 76) orientées différemment et un retardateur quart d'onde (74) disposé entre chacune des deux plaques biréfringentes (72, 76).

11. Le microscope selon l'une quelconque des revendications 9 ou 10, dans lequel le dispositif d'éclairage (9) comprend un réseau de miroirs pivotants individuels (60), chaque miroir (60) ayant une première et une deuxième position,
un prisme (50b) ayant des première, deuxième et troisième surfaces (62a, 62b, 62c), dans lequel, pour un miroir donné (60) dans la première position, la lumière provenant de la source de lumière (22) est réfléchie par le miroir donné (60), passe par la première surface (62a), est totalement réfléchie au niveau de la deuxième surface (62b) et sort par la troisième surface (62c), tandis que, pour le miroir donné (60) dans la deuxième position, la lumière provenant de la source de lumière (22) est réfléchie par le miroir donné (60), passe par la première surface (62a) et n'est pas totalement réfléchie au niveau de la deuxième surface (62b),
et dans lequel le séparateur de faisceau (70) est disposé à proximité dudit prisme (50b).

12. Le microscope selon la revendication 11, dans lequel le séparateur de faisceau (70) est disposé sur ladite première ou troisième surface (62a, 62c), en particulier ladite troisième surface (62c).

13. Le microscope selon la revendication 12, dans lequel le séparateur de faisceau (70) est relié à la première ou à la troisième surface (62a, 62c) par l'intermédiaire d'un matériau de remplissage transparent (71).

14. Le microscope selon l'une quelconque des revendications précédentes, dans lequel ledit filtre passe-bas (70, 90 - 100, 106) comprend un actionneur (90 - 100) adapté pour déplacer
au moins un élément optique de l'optique d'imagerie d'éclairage (26) et/ou
le modulateur spatial de lumière (24),
entre au moins une première et une deuxième position, générant ainsi, au niveau du plan objet (11), au moins deux images spatiales (82a - 82d), et dans lequel le microscope comprend une unité de commande (32) adaptée pour actionner périodiquement l'actionneur (90 - 100).

15. Le microscope selon la revendication 14, dans lequel l'optique d'imagerie d'éclairage (26) comprend au moins un miroir de déviation (28) déviant la lumière provenant du modulateur spatial de lumière (24) vers ledit plan objet (11), dans lequel l'actionneur (90) est adapté pour modifier au moins un angle d'inclinaison du miroir de déviation (28).

16. Le microscope selon l'une quelconque des revendications précédentes, dans lequel lesdites optiques d'éclairage ont un plan de Fourier (102) contenant une transformée de Fourier du modulateur spatial de lumière (24), dans lequel ledit filtre passe-bas (100) supprime les composantes de Fourier de l'image du modulateur spatial de lumière (24) dans le plan de Fourier (102).

17. Le microscope selon la revendication 16, dans lequel ledit filtre passe-bas (100) comprend plusieurs filtres sélectifs en longueur d'onde qui suppriment la fréquence spatiale fk et, de manière avantageuse, ses harmoniques, pour plusieurs longueurs d'onde différentes λi, les filtres comprenant des structures de blocage dimensionnées en fonction des différentes longueurs d'onde λi.

18. Le microscope selon la revendication 17, dans lequel le dispositif d'éclairage (9) comprend plusieurs sources de lumières (22a à 22d) ayant des spectres d'émission différents avec des émissions maximales aux longueurs d'onde λi.

19. Le microscope selon l'une quelconque des revendications précédentes,
dans lequel ledit dispositif d'éclairage (9) est pivotant autour d'un axe de pivotement (5) situé dans ledit plan objet (11) et
dans lequel le filtre passe-bas (70, 90 - 100, 106) est mis en œuvre par ladite optique d'imagerie d'éclairage (26) projetant l'image du modulateur spatial de lumière (24) dans un plan image (104) qui est décalé d'au moins 500 µm par rapport au plan objet (11).

20. Microscope selon la revendication 19, dans lequel le plan objet (11) est situé entre le dispositif de microscope (8) et le plan image (104).

21. Microscope selon l'une quelconque des revendications précédentes, dans lequel le filtre passe-bas (70, 90 - 100, 106) comprend un diffuseur (106), en particulier un diffuseur diffractif.
